(19) Europäisches Patentamt
European Patent Office
Office européen des brevets

(11) Veröffentlichungsnummer: **0 149 802**
**B1**

(12) # EUROPÄISCHE PATENTSCHRIFT

(45) Veröffentlichungstag der Patentschrift: **26.09.90**

(21) Anmeldenummer: **84115518.7**

(22) Anmeldetag: **15.12.84**

(51) Int. Cl.[5]: **C 07 D 241/42,** G 03 G 5/00,
C 07 D 241/38,
C 07 D 401/06,
C 07 D 403/06, C 07 D 405/06

(54) **Neue 2,3-Bis(Arylethenyl)-Chinoxaline, Verfahren zu Ihrer Herstellung und Ihre Verwendung als Photoleitfähige Verbindungen.**

(30) Priorität: **21.12.83 DE 3346177**

(43) Veröffentlichungstag der Anmeldung:
**31.07.85 Patentblatt 85/31**

(45) Bekanntmachung des Hinweises auf die Patenterteilung:
**26.09.90 Patentblatt 90/39**

(84) Benannte Vertragsstaaten:
**BE CH DE FR GB IT LI NL**

(56) Entgegenhaltungen:
**US-A-4 119 460**

**CHEMICAL ABSTRACTS, Band 72, 1970, Seite 396, Nr. 66896q, Columbus, Ohio, US; J. KLICNAR et al.: "Synthesis and photochemical effect of some substituted styrylquinoxalines on a photographic emulsion"**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Pawlowski, Georg, Dr.Dipl.Chem.**
**Blücherstrasse 48**
**D-6200 Wiesbaden (DE)**

(56) Entgegenhaltungen:

**CHEMICAL ABSTRACTS, Band 71, 1969, Seite 288, Nr. 38908f, Columbus, Ohio, US; N. SALDABOLS et al.: "Synthesis and antimicrobial action of alpha-2-(5-nitro-2-furyl)vinylr quinoxaline and its derivatives"**

**CHEMICAL ABSTRACTS, Band 102, 1985, Seite 554, Nr. 15024j, Columbus, Ohio, US; & JP - A - 59 45 444**

**EP 0 149 802 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft photoleitfähige 2,3-Bis(arylethenyl)-chinoxaline, ein Verfahren zu ihrer Herstellung und ihre Verwendung als photoleitfähige Verbindungen, zum Beispiel in der Elektrophotographie.

Obwohl seit langem bekannt, sind bisher in der Literatur nur wenige Verfahren zur Herstellung von 2,3-Bis(arylethenyl)-chinoxalinen und auch nur eine sehr geringe Anzahl von Derivaten dieser Verbindungsklasse bekannt geworden.

So wurden einige 2,3-Bis(aryl-ethenyl)-chinoxaline, wie beispielsweise 2,3-Di(p-nitrostyryl)-chinoxalin durch Umsetzung von 2,3-Dimethylchinoxalin mit p-Nitrobenzaldehyd in siedendem Essigsäureanhydrid als Kondensationsmittel erhalten (G. M. Bennet und G. H. Willis, J. Chem. Soc., *1928*, 1960; vgl. auch J. Klicnar, M. Vavra, P. Vetesnik und S. Panusowa, C. A. *72*, 66896).

2,3-Di(thienyl-2-vinyl)-chinoxalin wurde in geringer Ausbeute durch basisch katalysierte Umsetzung von 2,3-Dimethylchinoxalin mit 2-Thiophenaldehyd hergestellt (W. Ried und S. Hinsching, Ann. *600*, 47 (1956)).

2,3-Distyrylchinoxalin sowie einige seiner höheren Homologen wurden durch Umsetzungen von entsprechend substituierten 1,2-Diketonen mit o-Phenylendiamin unter saurer Katalyse erhalten (P. Karrer und C. Cochand, Helv. Chim. Acta, 28, 1181 (1945); vgl. auch H. Schlenk, Chem. Ber. *81*, 175 (1948)).

Die Herstellung aus der Literatur bekannter 2,3-Bis(arylethenyl)-chinoxaline erfolgte im wesentlichen nach diesen Herstellungsmethoden. Allen Umsetzungstypen ist jedoch gemeinsam, daß die zur Herstellung der ethylenischen Doppelbindung notwendigen Kondensationsreaktionen in technisch uninteressanten Ausbeuten stattfinden und auch nur bestimmte, geeignete aromatische Aldehyde Anwendung finden können.

Aus US—A 4,119,460 sind photoleitfähige Mischungen bekannt, die neben dem Photoleiter, wie p-Terphenyl, dispergiert in Cellulosenitraten, N-heteroaromatischen Verbindungen, wie Chinoxaline als Sensibilisatoren enthalten.

Es war Aufgabe der Erfindung photoleitfähige 2,3-Bis(aryl-ethenyl)-chinoxaline zur finden und diese durch ein universell anwendbares Verfahren, welches die Verbindungen in guten Ausbeuten bei niedrigen Kosten liefert, herzustellen.

Gegenstand der vorliegenden Erfindung sind photoleitfähige 2,3-Bis(aryl-ethenyl)-chinoxaline der allgemeinen Formel

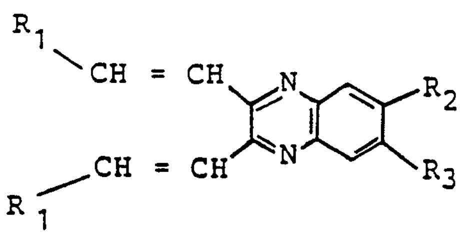

I

in der $R_1$ Phenyl oder Naphthyl, das durch mindestens einen substituienten aus der Reihe Halogen, Amino, Mono- oder Dialkylamino, Mono- oder Diarylamino, Alkyl, Alkenyl, Alkylendioxy, Azido, Halogenalkyl, Perfluoroalkyl mit jeweils 1 bis 4 Kohlenstoffatomen, Phenyl oder Styryl, Hydroxy, Alkoxy mit 1 oder 2 Kohlenstoffatomen, Phenoxy, Carboxy, Carboxyalkyl mit 1 bis 2 Kohlenstoffatomen, Carbamido, Cyano, Mercapto, Sulfonato oder Sulfonamido substituiert sein kann, Styryl, Anthracenyl, Phenanthrenyl, Chinolinyl, Carbazolyl, Furanyl, Pyrryl, Pyridyl oder Ferrocenyl, gegebenenfalls substituiert durch Halogen, Halogenalkyl, Alkyl, Alkylendioxy, Alkoxy, Carboxyalkyl mit jeweils 1 bis 4 Kohlenstoffatomen, Amino, Mono- oder Diallylamino mit jeweils 1 bis 4 Kohlenstoffatomen, Mono- oder Diarylamino, Cyano, Phenoxy oder Azido, oder Pyrenyl, Thienyl, Julolidyl, Benzofuranyl, Benzothienyl, Benzoxazolyl oder Benzimidazolyl, und

$R_2$ und $R_3$ gleich oder verschieden sind und Wasserstoff, Halogen, Amino, Mono- oder Dialkylamino, Alkyl, Alkoxy, Alkenyl mit jeweils 1 bis 4 Kohlenstoffatomen, Hydroxy, Carboxy, Carboalkoxy mit 1 bis 4 Kohlenstoffatomen oder Phenoxy oder beide zusammen einen ankondensierten, carbocyclischen aromatischen Ring darstellen, wobei solche Verbindungen der allgemeinen Formel I ausgenommen sind, in denen

$R_2$ und $R_3$ Wasserstoff,

$R_1$ Phenyl, Dibenzyl, Styrylphenyl, Styryl, p-Hydroxyphenyl, m- oder p-Methoxyphenyl-, 2,3- oder 3,4-Dimethoxyphenyl, 3,4,5-Trimethoxyphenyl, 3,4-Methylendioxyphenyl, o-, m- oder p-Chlorphenyl, o-Jodphenyl, p-Cyanophenyl, Furyl und Thienyl,

$R_2$ Methyl und $R_3$ Wasserstoff und

$R_1$ Phenyl, p-Methoxyphenyl, 3,4-Dimethoxyphenyl oder 3,4-Methylendioxyphenyl,

$R_2$ Amino oder Nitro und $R_3$ Wasserstoff und

$R_1$ Phenyl, p-Chlorphenyl, o- oder m-Hydroxyphenyl, m-Methoxyphenyl, 3,4-Dimethoxyphenyl oder 2-Hydroxy-1-naphthyl bedeuten.

Bevorzugt sind solche Verbindungen der allgemeinen Formel I, in der

$R_1$ Phenyl, Styryl, Naphtyl, Anthracenyl, Phenanthrenyl, Chinolinyl, Carbazolyl, Furanyl, Pyrryl, Pyridyl oder Ferrocenyl, gegebenenfalls substituiert durch Halogen, Halogenalkyl, Alkyl, Alkylendioxy, Alkoxy, Carboxyalkyl mit jeweils 1 bis 4 Kohlenstoffatomen, Amino, Mono- oder Dialkylamino mit jeweils 1 bis 4 Kohlenstoffatomen, Mono- oder Diarylamino, Cyano, Phenoxy oder Azido und

$R_2$ und $R_3$ gleich oder verschieden sind und Wasserstoff, Alkyl, Alkoxy mit 1 bis 4 Kohlenstoffatomen oder Halogen bedeuten.

Besonders bevorzugt sind Verbindungen der allgemeinen Formel I, in der

$R_1$ Aminophenyl, Dialkylaminophenyl mit 1 bis 4 Kohlenstoffatomen, Diarylaminophenyl, Cyanophenyl, Azidophenyl, Naphthyl, anthracenyl, Phenanthrenyl, Chinolinyl, n-Alkylcarbazolyl mit 1 bis 4 Kohlenstoffatomen oder Ferrocenyl und

$R_2$ und $R_3$ gleich oder verschieden sind und Wasserstoff, Methyl, Ethyl, Methoxy oder Halogen bedeuten.

Die erfindungsgemäßen Verbindungen der allgemeinen Formel I lassen sich herstellen, indem man ein gegebenenfalls substituiertes 2,3-Bis(dialkyloxyphosphono-methyl)-chinoxalin der allgemeinen Formel

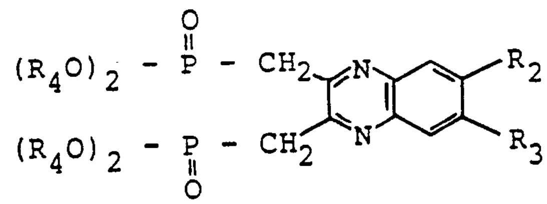

II

mit einem aromatischen oder nicht enolisierbaren Aldehyd der allgemeinen Formel

$$R_1—CHO$$

in denen $R_1$, $R_2$ und $R_3$ die angegebenen Bedeutungen haben und $R_4$ Alkyl mit 1 bis 4 Kohlenstoffatomen darstellt, in einem Lösungsmittel oder Lösungsmittelgemisch unter Zusatz eines basischen Kondensationsmittels im Temperaturbereich von 20 bis 160°C umsetzt, die ausgefallene Substanz abtrennt, gegebenenfalls reinigt und trocknet.

Die 2,3-Bis(dialkoxyphosphono-methyl)-chinoxaline der allgemeinen Formel II werden erhalten, indem man die entsprechenden 2,3-Bisbromomethylchinoxaline, welche analog zu bekannten Literaturvorschriften erhältlich sind (J. Wegmann und H. Dahn, Helv. Chim. Acta, *29*, 100 (1946)), mi einem Überschuß eines Phosphorigsäuretrialkylesters erhitzt.

Als 2,3-Bisbromomethylchinoxaline können beispielsweise verwendet werden: 2,3-Bisbromomethylchinoxalin, 2,3-Bisbromomethyl-6-methylchinoxalin, 2,3-Bisbromomethyl-6,7-dimethylchinoxalin, 2,3-Bisbromomethyl-6-methoxychinoxalin, 2,3-Bisbromomethyl-6-ethoxychinoxalin, 2,3-Bisbromomethyl-6-chlorchinoxalin oder 2,3-Bisbromomethyl-6,7-dichloro-chinoxalin.

Als Phosphorigsäuretrialkylester lassen sich beispielsweise verwenden: Phosphorigsäure-trimethylester, Phosphorigsäuretriethylester, Phosphorigsäuretripropylester, Phosphorigsäure-triisopropylester oder Phosphorigsäuretributylester.

Die dabei in sehr guten Ausbeuten erhältlichen 2,3-Bis-(dialkoxyphosphono-methyl)-chinoxaline der allgemeinen Formel II können gegebenenfalls durch Umkristallisation aus inerten, wenig polaren Lösungsmitteln gereinigt werden, lassen sich jedoch auch ohne weitere Aufarbeitung zur erfindungsgemäßen Umsetzung mit Aldehyden der allgemeinen Formel III verwenden.

Als Aldehyde der allgemeinen Formel III kommen in Frage: aromatische Aldehyde wie Chlorbenzaldehyde, Dialkylaminobenzaldehyde, Perfluorobenzaldehyde, Anisaldehyde, Phenoxybenzaldehyde, Carbomethoxybenzaldehyde, Toluolaldehyde, Naphthaldehyde, Pyrenaldehyde, Stilbenaldehyde, heterocyclische Aldehyde wie Thiophenaldehyde, Furfurale, Chinolinaldehyde, Pyridinaldehyde, Pyrimidinaldehyde, Carbazolaldehyde, aber auch andere, nicht enolisierbare Aldehyde. Diese Aldehyde sind nach zahlreichen bekannten Methoden zugänglich.

Die erfindungsgemäße Umsetzung der 2,3-Bis(dialkoxyphosphono-methyl)-chinoxaline der Formel II mit den Aldehyden der Formel III erfolgt bevorzugt in polarprotischen Lösungsmitteln oder Lösungsmittelgemischen. Brauchbare Lösungsmittel sind beispielsweise Alkohole, Etheralkohole oder substituierte Formamide. Als besonders bevorzugte Lösungsmittel seien Ethanol, Ethylenglykolmonomethylether oder Dimethylformamid erwähnt. Die Wahl des Lösungsmittels hängt dabei weitestgehend von den Löslichkeitseigenschaften der erfindungsgemäß eingesetzten Aldehyde nach der allgemeinen Formel III ab.

Als basische Kondensationsmittel werden bevorzugt Natriumhydroxid, Kaliumhydroxid, Natriumhydrid, Natriumamid, insbesondere aber Alkalialkoholate, wie Natriummethylat, Natriumethylat oder Kalium-t-butanolat verwendet.

Die Umsetzung der Reaktanden erfolgt vorzugsweise in einem Alkohol in Gegenwart eines Alkalialkoholates.

Die Stöchiometrie der eingesetzten Reaktanden kann in relativ weiten Grenzen variiert werden. Bevorzugt wird ein Verhältnis von Chinoxalin der allgemeinen Formel II zu Aldehyd der allgemeinen Formel III zu Base von etwa 1:2,1:5 angewendet, allerdings lassen sich sowohl der Aldehyd als auch die Base bei Verwendung bestimmter Reaktanden in höheren Konzentrationen einsetzen.

Das Verhältnis von Lösungsmittel zur Summe der Reaktanden kann erfindungsgemäß ebenfalls in einem sehr weiten Bereich variiert werden. Im allgemeinen wird so gearbeitet, daß ein Gewichtsverhältnis von 50:1 bis 10:1 von Lösungsmittel zur Summe von Phosphonomethylchinoxalin der allgemeinen Formel II und Aldehyd der allgemeinen Formel III Verwendung findet.

Das erfindungsgemäße Verfahren kann in einem relativ breiten Temperaturbereich von 20 bis 160°C durchgeführt werden, wobei bevorzugt der Temperaturbereich zwischen 60 und 120°C angestrebt wird.

Die Reaktionsdauer liegt je nach Art der Reaktanden im Bereich weniger Minuten bis einiger Stunden. Eine Reaktionsdauer von etwa 2 bis 3 Stunden ist jedoch bevorzugt.

Im allgemeinen wird das erfinungsgemäße Herstellungsverfahren so geführt, daß zunächst das basische Kondensationsmittel im Lösungsmittel vorgelegt, das Phosphonomethylchinoxalin der Formel II darin gelöst und bei Raumtemperatur eine Lösung des entsprechenden Aldehyds der Formel III rasch hinzugefügt wird. Das sich schnell verfärbende und erwärmende Gemisch wird dann unter Rühren auf die gewünschte Temperatur gebracht. Alternativ kann aber auch so gearbeitet werden, daß zunächst das Phosphonomethylchinoxalin der allgemeinen Formel II und der Aldehyd der allgemeinen Formel III in einer ausreichenden Menge des Lösungsmittels gelöst werden und dann die gelöst Base zugegeben wird.

Die so hergestellten erfindungsgemäßen 2,3-Bis(arylethenyl)-chinoxaline der allgemeinen Formel I fallen unter diesen Bedingungen oftmals schon aus der noch heißen Lösung so rein an, daß sich eine weitere Reinigung, beispielsweise durch Sublimation oder Umkristallisation, erübrigt. Für die Herstellung hochreiner Verbindungen ist jedoch die Umkristallisation aus einem wenig polaren Lösungsmittel günstig.

Wenn die erfindungsgemäßen Verbindungen der allgemeinen Formel I nicht oder nur unvollstängig aus dem Reaktionsgemisch auskristallisieren, so lassen sie sich durch Zugabe von etwa der doppelten Menge Wasser oder eines Nichtlösers (ggf. Neutralisation der Mischung) und anschließender Filtration oder Extraktion mit einem nicht mit Wasser mischbaren Lösungsmittel, z.B. Toluol, und anschließendem Abrotieren des Lösungsmittels ebenfalls sehr rein erhalten.

Nach beiden Methoden ist ide Ausbeute oft quantitativ.

Die erfindungsgemäßen neuen 2,3-Bis(arylethenyl)-chinoxaline der allgemeinen Formel I zeigen, insbesondere wenn es sich bei den Resten $R_1$ um Aminophenyl, Dialkylaminophenyl, Diarylaminophenyl, Cyanophenyl, Azidophenyl, Naphthyl, Anthracenyl, Phenanthrenyl, Chinolyl, N-Alkylcarbazolyl oder Ferrocenyl oder davon abgeleitete Reste handelt, sehr gute Photoleitereigenschaften. Sie Lassen sich daher gut als photoleitfähige Verbindungen zur Herstellung von photoleitfähigen Schichten in der Elektrophotographie verwenden.

Die nachstehenden Beispiele sollen die Erfindung erläutern. Prozente sind immer Gewichtsprozente, Schmelz- und Siedepunkte sind, sofern nicht anders angegeben, unkorrigiert.

Da die Herstellung von 2,3-Bis(dialkoxyphosphono-methyl)-chinoxalinen der Formel II, von denen in der Literatur bislang nur ein Vertreter beschrieben wurde (B. A. Arbusov und W. M. Soroastrowa, Izv. Akad. Nauk. SSSR, Otdel. Khim. Nauk., *1954*, 806), nach einer vereinfachten Vorschrift gelungen ist, wird im folgenden beispielhaft die Herstellung einer dieser erfindungsgemäßen Ausgangsverbindungen beschrieben.

### Beispiel 1

Herstellung von 2,3-Bis(diethoxyphosphono-methyl)-chinoxalin:

63,2 g (0,2 mol) 2,3-Bisbromomethylchinoxalin und 150 g (0,9 mol) Phosphorigsäuretriethylester werden in einem 1 1-Kolben, der über eine Destillationsbrücke mit einem Auffanggefäß verbunden ist, unter ständigem Rühren und Ausschluß der Luftfeuchtigkeit langsam erhitzt. Ab etwa 60°C Innentemperatur beginnt sich das Chinoxalin zu lösen und unter stark exothermer Reaktion, bei der sich die Mischung bis auf ca. 140°C erwärmt, destilliert abgespaltenes Ethylbromid in die Vorlage. Wenn der Hauptteil des Ethylbromids überdestilliert ist, wird mittels einer äußeren Heizquelle auf 140°C gehalten und die Reaktion 3 Stunden bei dieser Temperatur weitergerührt. Man läßt abkühlen und destilliert überschüssigen Phosphorigsäuretriethylester im Vakuum ab, letzte Reste werden durch Destillation im Hochvakuum und einer Innentemperatur bis ca. 100°C entfernt. Der Rückstand, ein rötlich-braunes Öl, welches beim Stehen kristallisiert, wird entweder ohne weitere Reinigung weiterverwendet oder zwecks Reinigung aus n-Hexan umkristallisiert.

Ausbeute: 79,5 g (92% d. Th.), fleischfarbene Kristalle.

Elementaranalyse: ber.: P 14,43 gef.: P 14,45 Fp.: 92 bis 93°C (n-Hexan).

### Beispiel 2

Herstellung von 2,3-Bis(m-cyanophenylethenyl)-chinoxalin:

Zu einer Alkoholatlösung, welche aus 1,95 g (0,085 mol) Natrium und 150 ml absolutem Ethanol hergestellt wurde, werden unter Rühren und Feuchtigkeitsausschluß 8,6 g (0,02 mol) der in Beispiel 1 hergestellten Verbindung hinzugefügt. Die rosafarbene Lösung wird mit einer Lösung von 5,9 g (0,045 mol)

m-Cyanobenzaldehyd in 30 ml absolutem Ethanol versetzt und zum Rückfluß erhitzt. Bereits nach wenigen Minuten beginnt die Kristallisation eines gelben Niederschlages von 2,3-Bis(m-cyanophenylethenyl)-chinoxalin. Die Mischung wird 2 Stunden am Rückfluß erhitzt und dann bis auf 40°C abkühlen gelassen. Das kanariengelbe Produkte wird abgenutscht und mit Ethanol gewaschen. Die Rohausbeute des getrockneten Produktes beträgt 6,65 g (87%). Es zeigt einen Schmelzpunkt von 200 bis 203°C. Zur Reinigung wird es aus einem 3:1-Gemisch aus Dimethylformamid und Wasser umkristallisiert.
Ausbeute: 5,9 g (77% d. Th.), kanariengelbe Kristalle.
$C_{26}H_{16}N_4$; MG.: 384,44

| | | | | |
|---|---|---|---|---|
| Elementaranalyse: | ber.: | C 81,25% | H 4,19% | N 14,57% |
| | gef.: | 80,8 % | 4,5 % | 14,2 % |

Fp.: 205°C
UV (MeOH): 295, 384 nm.

Beispiel 3

Herstellung von 2,3-Bis(p-dimethylaminophenylethenyl)chinoxalin:

1,95 g (0,085 mol) Natrium, 8,6 g (0,02 mol) der in Beispiel 1 hergestellten Verbindung und 6,7 g (0,045 mol) p-Dimethylaminobenzaldehyd werden, wie in Beispiel 2 beschrieben, miteinander umgesetzt. Aus der zunächst klaren, rötlichen Lösung kristallisieren beim Erhitzen auf Rückflußtemperatur rötliche Mikrokristalle. Nach ca. 3 Stunden wird die Lösung erkalten gelassen und zur vollständigen Kristallisation in den Kühlschrank gestellt. Nach Filtration werden orangefarbene Kristalle isoliert.
Ausbeute: 5,5 g (66% d. Th.), orangefarbene Mikrokristalle (Toluol).
$C_{28}H_{28}N_4$; MG.: 420,56

| | | | | |
|---|---|---|---|---|
| Elementaranalyse: | ber.: | C 79,97% | H 6,71% | N 13,32% |
| | gef.: | 79,4 % | 6,7 % | 13,2 % |

Fp.: 180 bis 181°C
UV (MeOH): 353, 434 nm.

Beispiel 4

Herstellung von 2,3-Bis(N-ethyl-carbazolyl-3-ethenyl)-chinoxalin:

Zu 120 ml Dimethylformamid werden unter Rühren und Feuchtigkeitsausschluß 9,0 g (0,08 mol) Kalium-t-butanolat hinzugefügt. Zu der entstandenen Lösung werden 8,6 g der in Beispiel 1 hergestellten Verbindung gegeben und die dunkelgrüne Lösung mit 9,0 g (0,04 mol) N-Ethyl-3-carbazolylaldehyd versetzt. Das schlagartig ausfallende gelbe Produkt geht beim Erhitzen auf Rückflußtemperatur wieder in Lösung. Nach 2,5 Stunden wird abgekühlt und die Mischung mit der gleichen Menge Ethanol versetzt, 10 Minuten gerührt, abgenutscht und getrocknet. Der bräunlich-gelbe Rückstand vom Fp. 250 bis 256°C wird aus wenig Dimethylformamid umkristallisiert.
Ausbeute: 10,3 g (92% d. Th.), intensiv gelbes Pulver.
$C_{40}H_{32}N_4$; MG.: 568,72

| | | | | |
|---|---|---|---|---|
| Elementaranalyse: | ber.: | C 84,47% | H 5,67% | N 9,85% |
| | gef.: | 84,5 % | 5,8 % | 9.7 % |

Fp.: 270 bis 271°C
UV (MeOH): 295, 400 nm.

Beispiel 5

Herstellung von 2,3-Bis(N-ethyl-carbazolyl-3-ethenyl)chinoxalin:

Zu einer Lösung von 8,6 g (0,02 mol) der in Beispiel 1 hergestellten Verbindung und 9,0 g (0,04 mol) N-Ethyl-3-carbazolylaldehyd in 350 ml absolutem Ethanol wird unter Rühren und Erwärmen auf 60°C eine Lösung von 1,95 g (0,08 mol) Natrium in 100 ml absolutem Ethanol zugetropft. An der Eintropfstelle beginnt sofort die Ausfällung eines kanariengelben Produktes. Nach beendeter Zugabe wird 3 Stunden zum Rückfluß erhitzt und heiß filtriert. Der intensiv gelbe Rückstand vom Fp. 269°C wird nach Trocknen aus Dimethylformamid umkristallisiert.
Ausbeute: 9,6 g (87% d. Th.)
Fp.: 271°C.

Beispiele 6 bis 77

Die nachstehend beschriebenen Verbindungen wurden analog der Umsetzung in Beispiel 2 hergestellt, wobei lediglich geringfügige Änderungen der Reaktionsdauer, des Verhältnisses Reaktanden zu

Lösungsmittel und der Aufarbeitung durchgeführt wurden. Die mit einem Stern versehenen Beispiele (*) wurden analog zu Beispiel 4 hergestellt.

| Beispiel Nr. | $R_1$ | $R_2$ | $R_3$ | Fp. | |
|---|---|---|---|---|---|
| 6 | 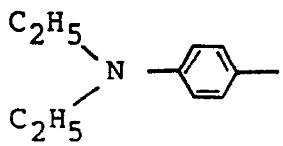 | H | H | 193-194 | Toluol/ Benzin |
| 7 | 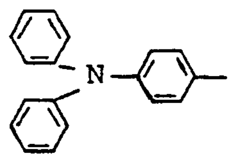 | H | H | 197-198 | Toluol |
| 8 | 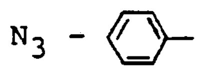 | H | H | 165 (Z.) | Toluol |
| 9 | 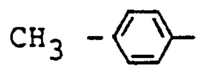 | H | H | 184-185 | Benzin |
| 10 | 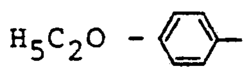 | H | H | 181-182 | Toluol/ Benzin |
| 11 | 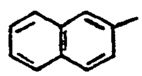 | H | H | 217-218 | m-Xylol |
| 12 | 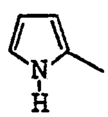 | H | H | 225-226 | m-Xylol |
| 13 | 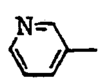 | H | H | 179 | Toluol/ Benzin |
| 14 | 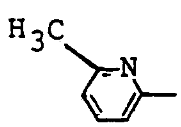 | H | H | 180-181 | Toluol/ Benzin |
| 15 | 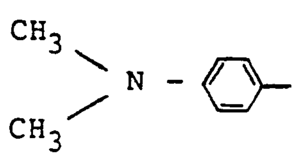 | $CH_3$ | H | 162 | DMF |
| 16 | 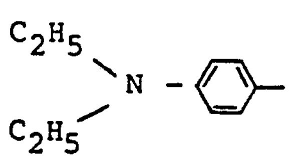 | $CH_3$ | H | 150-152 | EtOH |

| Beispiel Nr. | $R_1$ | $R_2$ | $R_3$ | Fp. | |
|---|---|---|---|---|---|
| 17 | 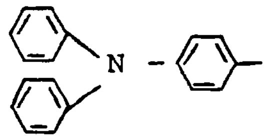 | $CH_3$ | H | 178-179 | $CHCl_3$/ EtOH |
| 18 * | 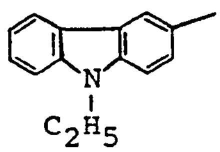 | $CH_3$ | H | 231-232 | DMF |
| 19 | 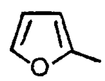 | $CH_3$ | H | 153 | Toluol |
| 20 | 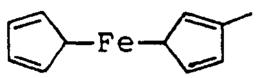 | $CH_3$ | H | 217-218 | Toluol |
| 21 * | 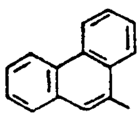 | $CH_3$ | H | > 260 | DMF |
| 22 | 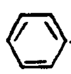–CH = CH - | $CH_3$ | H | 174-175 | EtOH |
| 23 | $CH_3$–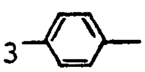 | $CH_3$ | H | 162-163 | Aceton/ $H_2O$ |
| 24 | 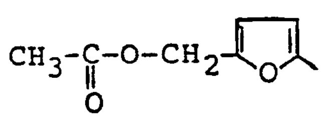 | $CH_3$ | H | 198-199 | $CHCl_3$/ EtOH |
| 25 | 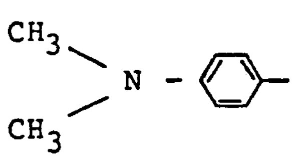 | $OCH_3$ | H | 166-167 | $CHCl_3$/ EtOH |
| 26 | 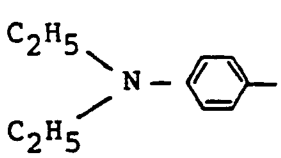 | $OCH_3$ | H | 155-156 | $CHCl_3$/ EtOH |

| Beispiel Nr. | $R_1$ | $R_2$ | $R_3$ | Fp. | |
|---|---|---|---|---|---|
| 27 | 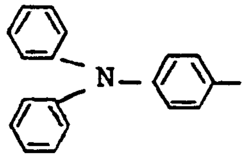 | $OCH_3$ | H | 245-246 | Toluol |
| 28 | 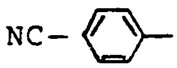 | $OCH_3$ | H | 255-256 | $CHCl_3$/ EtOH |
| 29 | 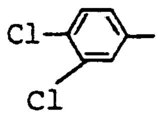 | $OCH_3$ | H | 215 | DMF |
| 30 | 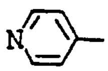 | $OCH_3$ | H | 253-254 | DMF/$H_2O$ |
| 31 | 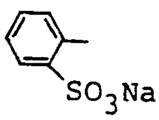 | $OCH_3$ | H | >260 | |
| 32 * | 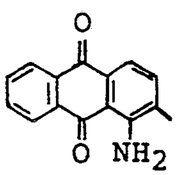 | $OCH_3$ | H | >260 | DMF |
| 36 | 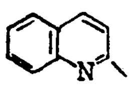 | $OCH_3$ | H | 204-205 | Toluol |
| 37 | 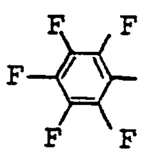 | $OC_2H_5$ | H | 169-170 | $CHCl_3$/ DMF |
| 38 | 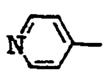 | $OC_2H_5$ | H | 202-203 | $CHCl_3$/ EtOH |
| 39 | 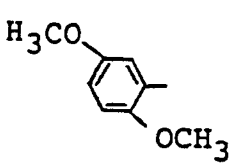 | $OC_2H_5$ | H | 125-126 | $CHCl_3$/ EtOH |

| Beispiel Nr. | $R_1$ | $R_2$ | $R_3$ | Fp. | |
|---|---|---|---|---|---|
| 40 | 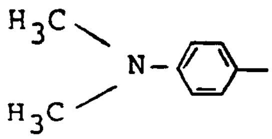 | $OC_2H_5$ | H | 205-206 | DMF/$H_2O$ |
| 41 | 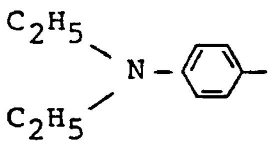 | $OC_2H_5$ | H | 161-162 | $CHCl_3$/ EtOH |
| 42 | 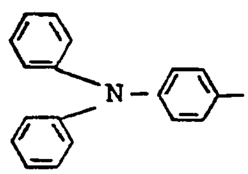 | $OC_2H_5$ | H | 239-240 | Toluol |
| 43* | 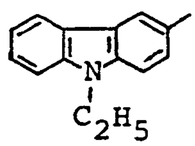 | $OC_2H_5$ | H | > 260 | DMF |
| 44 | 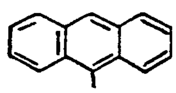 | $OC_2H_5$ | H | 242-243 | $CHCl_3$ |
| 45 | 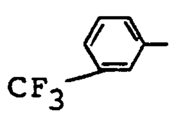 | $OC_2H_5$ | H | 155-156 | $CHCl_3$/ EtOH |
| 46 | 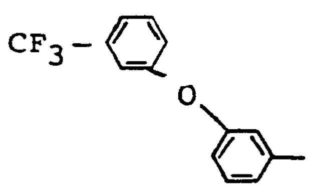 | $OC_2H_5$ | H | 97- 98 | $CHCl_3$/ EtOH |
| 47 | 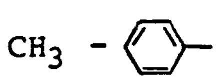 | $OC_2H_5$ | H | 173-174 | $CHCl_3$/ EtOH |
| 48 | 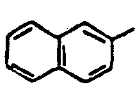 | $OC_2H_5$ | H | 179-180 | $CHCl_3$/ EtOH |
| 49 | 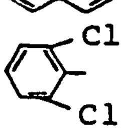 | $OC_2H_5$ | H | 195-196 | $CHCl_3$ |
| 50 | 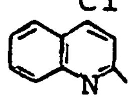 | $OC_2H_5$ | H | 211-212 | $CHCl_3$/ EtOH |

| Beispiel Nr. | $R_1$ | $R_2$ | $R_3$ | Fp. | |
|---|---|---|---|---|---|
| 51 | 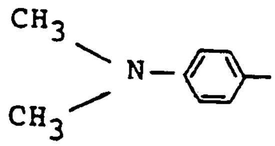 | 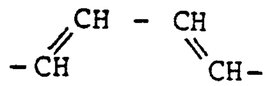 | | 217-218 | Toluol |
| 52 | 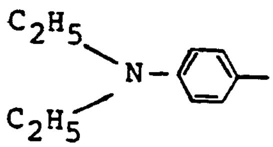 | 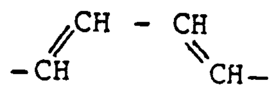 | | 215-216 | Toluol |
| 53 | 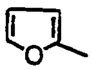 | 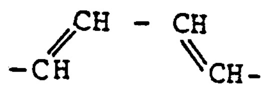 | | 230-231 | Toluol |
| 54 | 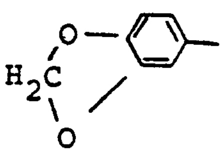 | 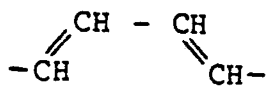 | | 249-250 | Toluol |
| 55 | 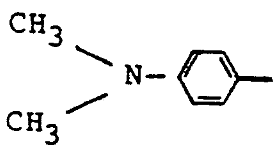 | $CH_3$ | $CH_3$ | 223-224 | $CHCl_3$/ EtOH |
| 56 | 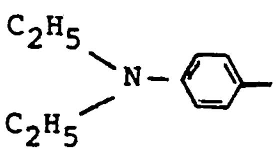 | $CH_3$ | $CH_3$ | 161-162 | $CHCl_3$/ EtOH |
| 57 | 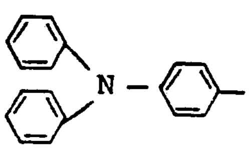 | $CH_3$ | $CH_3$ | 196-197 | $CHCl_3$/ EtOH |
| 58* | 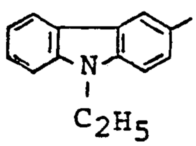 | $CH_3$ | $CH_3$ | 255-256 | DMF |
| 59 | 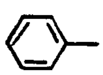 | $CH_3$ | $CH_3$ | 211-212 | $CHCl_3$/ EtOH |
| 60 | 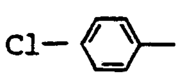 | $CH_3$ | $CH_3$ | 210 | $CHCl_3$/ EtOH |

| Beispiel Nr. | $R_1$ | $R_2$ | $R_3$ | Fp. | |
|---|---|---|---|---|---|
| 61 | 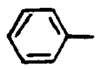–CH=CH– | $CH_3$ | $CH_3$ | 224–225 | $CHCl_3$/ EtOH |
| 62 | 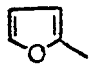 | $CH_3$ | $CH_3$ | 203–204 | $CHCl_3$/ EtOH |
| 63 | 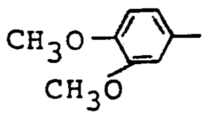 | $CH_3$ | $CH_3$ | 204–205 | $CHCl_3$/ EtOH |
| 64 | 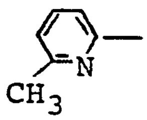 | $CH_3$ | $CH_3$ | 210–212 | EtOH |
| 65 | 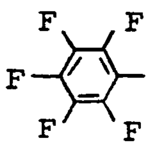 | $CH_3$ | $CH_3$ | 200–201 | $CHCl_3$/ EtOH |
| 66 | 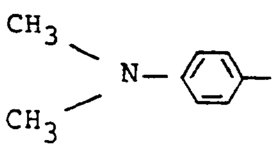 | Cl | H | 188–189 | $CHCl_3$/ EtOH |
| 67 | 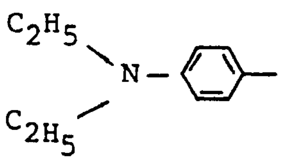 | Cl | H | 151–153 | $CHCl_3$/ EtOH |
| 68* | 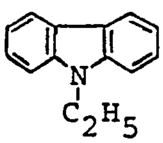 | Cl | H | 256–257 | DMF |
| 69 | 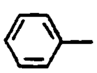 | Cl | H | 182–183 | $CHCl_3$/ EtOH |
| 70 | 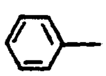– CH=CH– | Cl | H | 215 | $CHCl_3$/ EtOH |
| 71 | 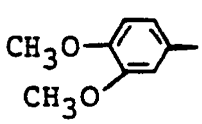 | Cl | H | 186–187 | $CHCl_3$/ EtOH |

| Beispiel Nr. | $R_1$ | $R_2$ | $R_3$ | Fp. | |
|---|---|---|---|---|---|
| 72 | 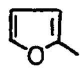 | Cl | H | 172-173 | $CHCl_3$/ EtOH |
| 73 | 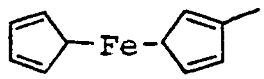 | Cl | H | 249-250 | DMF/ EtOH |
| 74 | 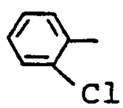 | Cl | H | 183-184 | $CHCl_3$/ EtOH |
| 75 | 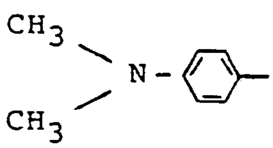 | Cl | Cl | 215 | Toluol |
| 76 | 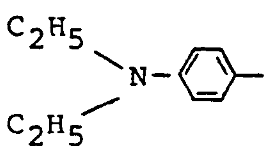 | Cl | Cl | 169-170 | Heptan |
| 77 | 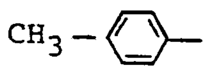 | Cl | Cl | 215-216 | Toluol |

Beispiel 78

Herstellung von 2,3-Bis(3,4-dimethoxyphenylethenyl)-6,7-dichlorchinoxalin:

Zu einer Lösung von 10 g (0,02 mol) 2,3-Bis(diethoxyphosphono-methyl)-6,7-dichlorchinoxalin, welches durch Umsetzung von 2,3-Bisbromomethyl-6,7-dichlorchinoxalin und Triethylphosphit erhalten wurde, und 6,75 g (0,045 mol) 3,4-Dimethoxybenzaldehyd in 180 ml Ethylenglykolmonomethylether wird unter Rühren eine Lösung von 1,95 g (0,08 mol) Natrium in 120 ml Ethylenglykolmonoethylether zugetropft. Die Lösung beginnt sich zu verfärben und nach wenigen Minuten fällt ein gelbes Produkt aus. Die Mischung wird 2 Stunden auf Rückflußtemperatur erhitzt und dann abgekühlt. Nachdem das Reaktionsgemisch über Nacht im Kühlschrank gelagert wurde, wird filtriert, der Rückstand mit viel Ethanol gewaschen und getrocknet. Man erhält ein gelbes Pulver in einer Ausbeute von etwa 10 g mit einem Schmelzpunkt von 203 bis 205°C. Man kristallisiert aus Toluol um.

Ausbeute: 8,7 g (83% d. Th.), intensiv gelbe Nadeln.

$C_{28}H_{24}N_2O_4Cl_2$; MG.: 523,41

| | | | | | |
|---|---|---|---|---|---|
| Elementaranalyse: | ber.: | C 64,25 | H 4,62 | N 5,35 | Cl 13,55 |
| | gef.: | 63,9 | 4,6 | 5,5 | 13,8 |

Fp.: 205—206°C

UV (MeOH): 334, 385, 424 nm.

Beispiel 79

Zur Herstellung einer Flachdruckform wird auf eine 0,3 mm starke, elektrochemisch aufgerauhte und mit Polyvinylphosphonsäure nachbehandelte Aluminiumfolie eine Beschichtungslösung, bestehend aus

3,3 g eines Copolymeren aus Styrol und Maleinsäureanhydrid mit einem mittleren Molekulargewicht von 80.000,
2,2 g der Verbindung aus Beispiel Nr. 17,
0,1 g Rhodamin FB (C.I. 45170),
0,6 g Astrazonorange (C.I. 48040) in
22,0 g Tetrahydrofuran und
18,8 g Methylglykolmonomethylether,

mittels einer Rakel aufgetragen und zu einer Schichtdicke von 5,6 getrocknet.

Die Schicht wird mit einer Corona auf −450 V aufgeladen und in einer Reprokamera mit 10 Halogenstrahlern zu je 600 Watt 16 Sekunden lang belichtet. Als Vorlage dient eine Klebemontage, die die üblichen Prüfelemente enthält.

Nach Trockenbetonerung und thermischer Fixierung erhält man ein saußeres, randscharfes Bild der Vorlage. Nach Entschichtung mittels eines Entschichters, welcher hergestellt wurde aus 50 g $Na_2SiO_3 \cdot 9H_2O$ in 250 g 85 %igen Glycerin, 390 g Ethylenglykol und 310 g Methanol, erhält man eine fertige Druckplatte, die mehrere tausend guter Drucke liefert.

Beispiel 80

Das in Beispiel 79 beschriebene Aufzeichnungsmaterial wird in einem Dyntest-Gerät vermessen. Die in diesem Gerät verwendete Glühfadenlampe hat eine Temperatur von 2800 K. die Schicht, welche bis auf ca. −950 V aufladbar ist, wird auf −500 V aufgeladen und die Dunkelentladung aufgezeichnet. Nach 60 sec beträgt das Restpotential $U_D$ −426 V = 85,2%. Wird die Schicht auf −500 V aufgeladen und mittels obiger Lampe belichtet, so beträgt das Restpotential $U_H$ nach 60 sec 15 V = 3,0%. Die Halbwertsempfindlichkeit $E_{1/2}$ beträgt etwa 18 J/cm$^2$.

Beispiel 81

In der in Beispiel 79 angegebenen Beschichtungslösung wird die Verbindung des Beispiels Nr. 17 durch die Verbindung des Beispiels Nr. 56 ersetzt. Die weitere Verarbeitung erfolgt, wie in Beispiel 79 beschrieben. Die erhaltene Schicht wird entsprechend Beispiel 80 vermessen. Das Material zeigt eine Halbwertsempfindlichkeit $E_{1/2}$ von 12 µJ/cm$^2$.

**Patentansprüche**

1. Photoleitfähige 2,3-Bis(arylethenyl)-chinoxaline der allgemeinen Formel

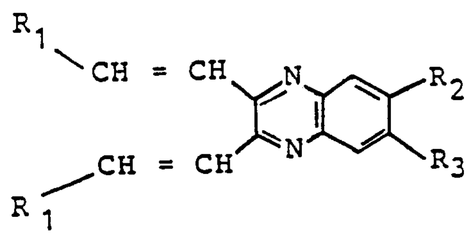

I

in der $R_1$ Phenyl oder Napthyl, das durch mindestens einen substituenten aus der Reihe Halogen, Amino, Mono- oder Dialkylamino, Mono- oder Diarylamino, Alkyl, Alkenyl, Alkylendioxy, Azido, Halogenalkyl, Perfluoroalkyl mit jeweils 1 bis 4 Kohlenstoffatomen, Phenyl oder Styryl, Hydroxy, Alkoxy mit 1 oder 2 Kohlenstoffatomen, Phenoxy, Carboxy, Carboxyalkyl mit 1 bis 2 Kohlenstoffatomen, Carbamido, Cyano, Mercapto, Sulfonato oder sulfonamido substituiert sein kann, Styryl, Anthracenyl, Phenanthrenyl, Chinolinyl, Carbazolyl, Furanyl, Pyrryl, Pyridyl oder Ferrocenyl, gegebenenfalls substituiert durch Halogen, Halogenalkyl, Alkyl, Alkylendioxy, Alkoxy, Carboxyalkyl mit jeweils 1 bis 4 Kohlenstoffatomen, Amino, Mono- oder Dialkylamino mit jeweils 1 bis 4 Kohlenstoffatomen, Mono- oder Diarylamino, Cyano, Phenoxy oder Azido, oder Pyrenyl, Thienyl, Julolidyl, Benzofuranyl, Benzothienyl, Benzoxazolyl oder Benzimidazolyl, und

$R_2$ und $R_3$ gleich oder verschieden sind und Wasserstoff, Halogen, Amino, Mono- oder Dialkylamino, Alkyl, Alkoxy, Alkenyl mit jeweils 1 bis 4 Kohlenstoffatomen, Hydroxy, Carboxy, Carboalkoxy mit 1 bis 4 Kohlenstoffatomen oder Phenoxy oder beide zusammen einen ankondensierten, carbocyclischen aromatischen Ring darstellen, wobei solche Verbindungen der allgemeinen Formel I ausgenommen sind, in denen

$R_2$ und $R_3$ Wasserstoff,

$R_1$ Phenyl, Dibenzyl, Styrylphenyl, Styryl, p-Hydroxyphenyl, m- oder p-Methoxyphenyl-, 2,3- oder 3,4-Dimethoxyphenyl, 3,4,5-Trimethoxyphenyl, 3,4-Methylendioxyphenyl, o-, m- oder p-Chlorphenyl, o-Jodphenyl, p-Cyanophenyl, Furyl und Thienyl,

$R_2$ Methyl und $R_3$ Wasserstoff und

$R_1$ Phenyl, p-Methoxyphenyl, 3,4-Dimethoxyphenyl oder 3,4-Methylendioxyphenyl,
$R_2$ Amino oder Nitro und $R_3$ Wasserstoff und
$R_1$ Phenyl, p-Chlorphenyl, o- oder m-Hydroxyphenyl, m-Methoxyphenyl, 3,4-Dimethoxyphenyl oder 2-Hydroxy-1-naphthyl bedeuten.

2. Verbindungen der allgemeinen Formel I nach Anspruch 1, in der $R_1$ Phenyl, Styryl, Naphtyl, Anthracenyl, Phenanthrenyl, Chinolinyl, Carbazolyl, Furanyl, Pyrryl, Pyridyl oder Ferrocenyl, gegebenenfalls substituiert durch Halogen, Halogenalkyl, Alkyl, Alkylendioxy, Alkoxy, Carboxyalkyl mit jeweils 1 bis 4 Kohlenstoffatomen, Amino, Mono- oder Dialkylamino mit jeweils 1 bis 4 Kohlenstoffatomen, Mono- oder Diarylamino, Cyano, Phenoxy oder Azido und
$R_2$ und $R_3$ gleich oder verschieden sind und Wasserstoff, Alkyl, Alkoxy mit 1 bis 4 Kohlenstoffatomen oder Halogen bedeuten.

3. Verbindungen der allgemeinen Formel I nach Anspruch 1,
in der $R_1$ Aminophenyl, Dialkylaminophenyl mit 1 bis 4 Kohlenstoffatomen, Diarylaminophenyl, Cyanophenyl, Azidophenyl, Naphthyl, Anthracenyl, Phenanthrenyl, Chinolinyl, N-Alkylcarbazolyl mit 1 bis 4 Kohlenstoffatomen oder Ferrocenyl und
$R_2$ und $R_3$ gleich oder verschieden sind und Wasserstoff, Methyl, Ethyl, Methoxy, Ethoxy oder Halogen bedeuten.

4. Verfahren zur Herstellung der Verbindungen gemäß Ansprüchen 1 bis 3, dadurch gekennzeichnet, daß man eine gegebenenfalls substituiertes 2,3-Bis-(dialkyloxyphosphono-methyl)-chinoxalin der allgemeinen Formel

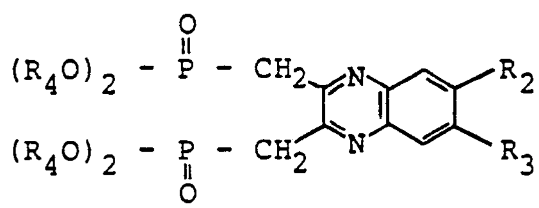

II

mit einem aromatischen oder nicht enolisierbaren Aldehyd der allgemeinen Formel

$$R_1—CHO$$

in denen $R_1$, $R_2$ und $R_3$ die angegebenen Bedeutungen haben und $R_4$ Alkyl mit 1 bis 4 Kohlenstoffatomen darstellt, in einem Lösungsmittel oder Lösungsmittelgemisch unter Zusatz eines basischen Kondensationsmittels im Temperaturbereich von 20 bis 160°C umsetzt, die ausgefallene Substanz abtrennt, gegebenenfalls reinigt und trocknet.

5. Verfahren nach Anspruch 4, dadurch gekennzeichnet, daß man die Umsetzung in Ethanol, Ethylenglykolmonomethylether oder Dimethylformamid durchführt.

6. Verfahren nach Anspruch 4 und 5, dadurch gekennzeichnet, daß man die Umsetzung der Reaktanden in einem Alkohol in Gegenwart eines Alkalialkoholates durchführt.

7. Verwendung der 2,3-Bis(arylethenyl)-chinoxaline nach Anspruch 1 als photoleitfähige Verbindungen zur Herstellung von photoleitfähigen Schichten.

**Revendications**

1. 2,3-bis(aryléthényl)-quinoxalines photoconductrices de formule générale

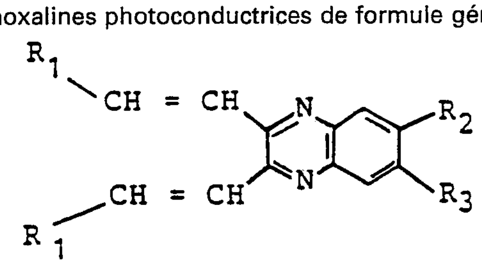

I

dans laquelle
$R_1$ représente un radical phényle ou naphtyle qui peut être substitué par au moins un substituant choisi parmi des halogènes et des groupes amino, mono- ou dialkylamino, mono- ou diarylamino, alkyle, alcényle, alkylènedioxy, azido, halogénoalkyle, perfluoroalkyle ayant chacun de 1 à 4 atomes de carbone, phényle ou styryle, hydroxy, alcoxy ayant 1 ou 2 atomes de carbone, phénoxy, carboxy, carboxyalkyle ayant de 1 à 4 atomes de carbone, carbamido cyano, mercapto, sulfonato ou sulfonamido, un radical styryle, anthracényle, phénanthrényle, quinolinyle, carbazolyle, furannyle, pyrryle, pyridyle ou ferrocényle, éventuellement substitués par des halogènes ou des groupes halogénoalkyle, alkyle, alkylènedioxy, alcoxy, carboxyalkyle ayant chacun de 1 à 4 atomes de carbone, amino, mono- ou dialkylamino ayant

chacun de 1 à 4 atomes de carbone, mono- ou diarylamino, cyano, phénoxy ou azido, ou un radical pyrényle, thiényle, julolidyle, Benzofurannyle, benzothiényle, benzoxazolyle ou benzimidazolyle, et

$R_2$ et $R_3$ sont identiques ou différents et représentent un atome d'hydrogène ou d'halogène ou un groupe amino, mono- ou dialkylamino, alkyle, alcoxy, alcényle ayant chacun de 1 à 4 atomes de carbone, hydroxy, carboxy, carboalcoxy ayant de 1 à 4 atomes de carbone, ou phénoxy, ou forment ensemble un noyau aromatique carbocyclique fusionné, à l'exclusion des composés de formule générale I dans lesquels

$R_2$ et $R_3$ représentent des atomes d'hydrogène,

$R_1$ représente le groupe phényle, dibenzyle, styrylphényle, styryle, p-hydroxyphényle, m- ou p-méthoxyphényle, 2,3- ou 3,4-diméthoxyphényle, 3,4,5-triméthoxyphényle, 3,4-méthylènedioxyphényle, o-, m- ou p-chlorophényle, o-iodophényle, p-cyanophényle, furyle ou thiényle;

$R_2$ représente le groupe méthyle et $R_3$ représente un atome d'hydrogène, et

$R_1$ représente le groupe phényle, p-méthoxyphényle, 3,4-diméthoxyphényle ou 3,4-méthylènedioxyphényle;

$R_2$ représente le groupe amino ou nitro et $R_3$ représente un atome d'hydrogène, et

$R_1$ représente le groupe phényle, p-chlorophényle, o- ou m-hydroxyphényle, m-méthoxyphényle, 3,4-diméthoxyphényle ou 2-hydroxy-1-naphtyle.

2. Composés de formule générale I selon la revendication 1, dans laquelle

$R_1$ représente un radical phényle, styryle, naphtyle, anthracényle, phénanthrényle, quinolinyle, carbazolyle, furannyle, pyrryle, pyridyle ou ferrocényle, éventuellement substitués par des halogènes ou par des groupes halogénoalkyle, alkyle, alkylènedioxy alcoxy, carboxyalkyle ayant chacun de 1 à 4 atomes de carbone, amino, mono- ou dialkylamino ayant chacun de 1 à 4 atomes de carbone, mono- ou diarylamino, cyano, phénoxy ou azido; et

$R_2$ et $R_3$ sont identiques ou différents et représentent un aotme d'hydrogène ou d'halogène ou un groupe alkyle ou alcoxy ayant de 1 à 4 atomes de carbone.

3. Composés de formule générale I selon la revendication 1, dans laquelle

$R_1$ représente un groupe aminophényle, dialkylaminophényle ayant de 1 à 4 atomes de carbone, dans chaque partie alkyle, diarylaminophényle, cyanophényle, azidophényle, naphtyle, anthracényle, phénanthrényle, quinolinyle, n-alkylcarbazoyle ayant de 1 à 4 atomes de carbone ou ferrocényle; et

$R_2$ et $R_3$ sont identiques ou différents et représentent un atome d'hydrogène ou d'halogène ou le groupe méthyle, éthyle, méthoxy ou éthoxy.

4. Procédé pour la préparation des composés selon les revendications 1 à 3, caractérisé en ce que l'on fait réagir une 2,3-bis- (dialkyloxyphosphono-méthyl)-quinoxaline éventuellement substituée, de formule générale

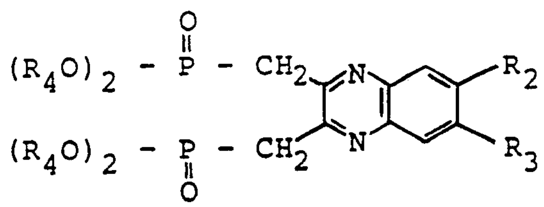

II

avec un aldéhyde aromatique ou non énolisable de formule générale

$R_1—CHO$

dans lesquelles formules $R_1$, $R_2$ et $R_3$ ont les significations données et $R_4$ représente un groupe alkyle ayant de 1 à 4 atomes de carbone, dans un solvent ou mélange de solvants, avec addition d'un agent basique de condensation à une température de 20 à 160°C, et en ce que la substance précipitée est séparée, éventuellement purifiée, et séchangée.

5. Procédé selon la revendication 4, caractérisé en ce que l'on effectue la réaction dans de l'éthanol, de l'éther monométhylique d'éthylèneglycol ou du diméthylformamide.

6. Procédé selon la revendications 4 et 5, caractérisé en ce que l'on effectue la réaction des partenaires réactionnels dans un alcool, en présence d'un alcoolate de métal alcalin.

7. Utilisation des 2,3-bis(aryléthényl)-quinoxalines selon la revendication 1, en tant que composés photoconducteurs pour la production de couches photoconductrices.

## Claims

1. Photoconductive 2,3-bis(arylethenyl)quinoxalines of the formula

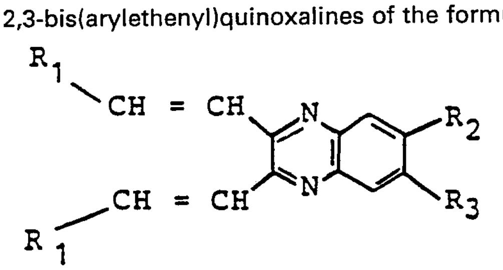

I

in which

$R_1$ denotes phenyl or naphthyl, which can be substituted by at least one substituent from the series comprising halogen, amino, monoalkylamino, dialkylamino, monoarylamino, diarylamino, alkyl, alkenyl, alkylenedioxy, azido, halogenoalkyl, perfluoroalkyl having 1 to 4 carbon atoms each, phenyl or styryl, hydroxy, alkoxy having 1 or 2 carbon atoms, phenoxy, carboxy, carboxyalkyl having 1 to 4 carbon atoms, carbamido, cyano, mercapto, sulfonato and sulfonamido; styryl, anthracenyl, phenanthrenyl, quinolinyl, carbazolyl, furanyl, pyrryl, pyridyl or ferrocenyl, which can be substituted by halogen, halogenoalkyl, alkyl, alkylenedioxy, alkoxy, carboxyalkyl having 1 to 4 carbon atoms each, amino, monoalkylamino, dialkylamino having 1 to 4 carbon atoms each, monoarylamino, diarylamino, cyano, phenoxy or azido; or pyrenyl, thienyl, julolidyl, benzofuranyl, benzothienyl, benzoxazolyl or benzimidazolyl; and

$R_2$ and $R_3$ are identical or different and each denote hydrogen, halogen, amino, monoalkylamino, dialkylamino, alkyl, alkoxy, alkenyl having 1 to 4 carbon atoms each, hydroxyl, carboxyl, carboalkoxy having 1 to 4 carbon atoms or phenoxy; or together represent a fused-on, carboxylic aromatic ring, except for compounds of formula I in which

$R_2$ and $R_3$ each denote hydrogen, and

$R_1$ denotes phenyl, dibenzyl, styrylphenyl, styryl, p-hydroxyphenyl, m- or p-methoxyphenyl, 2,3- or 3,4-dimethoxyphenyl, 3,4,5-trimethoxyphenyl, 3,4-methylenedioxyphenyl, o-, m- or p-chlorophenyl, o- iodophenyl, p-cyanophenyl, furfuryl or thienyl;

$R_2$ denotes methyl and $R_3$ denotes hydrogen and

$R_1$ denotes phenyl, p-methoxyphenyl, 3,4-dimethoxyphenyl or 3,4-methylenedioxyphenyl;

$R_2$ denotes amido or nitro and $R_3$ denotes hydrogen and

$R_1$ denotes phenyl, p-chlorophenyl, o- or m-hydroxyphenyl, m-methoxyphenyl, 3,4-dimethoxyphenyl or 2-hydroxy-1-naphthyl.

2. Compounds of the formula I in claim 1, in which

$R_1$ denotes phenyl, styryl, naphthyl, anthracenyl, phenanthrenyl, quinolinyl, carbazolyl, furanyl, pyrryl, pyridyl or ferrocenyl, which can be substituted by halogen, halogenoalkyl, alkyl, alkylenedioxy, alkoxy, carboxyalkyl having 1 to 4 carbon atoms each, amino, monoalkylamino, dialkylamino having 1 to 4 carbon atoms each, monoarylamino, diarylamino, cyano, phenoxy or azido and

$R_2$ and $R_3$ are identical or different and each denotes hydrogen, alkyl, alkoxy having 1 to 4 carbon atoms or halogen.

3. Compounds of the formula I in claim 1, in which

$R_1$ denotes aminophenyl, dialkylaminophenyl having 1 to 4 carbon atoms, diarylaminophenyl, cyanophenyl, azidophenyl, naphthyl, anthracenyl, phenanthrenyl, quinolinyl, n-alkylcarbazolyl having 1 to 4 carbon atoms or ferrocenyl and

$R_2$ and $R_3$ are identical or different and each denotes hydrogen, methyl, ethyl, methoxy, ethoxy or halogen.

4. A process for preparing the compounds as claimed in claims 1 to 3, characterized in that a substituted or unsubstituted 2,3-bis(dialkoxyphosphonomethyl)quinoxaline of the formula

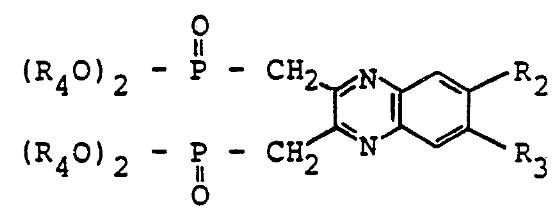

II

is reacted with an aromatic or non-enolizable aldehyde of the formula

$R_1$—CHO

$R_1$, $R_2$ and $R_3$ being as defined above and $R_4$ representing alkyl of 1 to 4 carbon atoms, within the temperature range from 20 to 160°C, in a solvent mixture in the presence of basic condensing agent, and that the precipitated substance is separated off, optionally purified, and dried.

5. The process as claimed in claim 4, characterized in that the reaction is carried out in ethanol, ethylene glycol monomethyl ether or dimethylformamide.

6. The process as claimed in claims 4 and 5, characterized in that the reactants are reacted in an alcohol in the presence of an alkali metal alcoholate.

7. Use of the 2,3-bis(arylethenyl)quinoxalines claimed in claim 1 as photoconductive compounds in the manufacture of photoconductive layers.